# EUROPEAN PATENT APPLICATION

(11) **EP 4 552 479 A1**
(43) Date of publication of application: **14.05.2025**
(21) Application number: 23835533.3
(22) Date of filing: 04.07.2023
(51) Int. Cl.: A01G 7/00, G01N 21/27

(54) **SUPPORT STRUCTURE AND SYSTEM**

(30) Priority: 06.07.2022 JP 2022109088
(71) Applicant: Tohoku University, Sendai-shi, Miyagi 980-8577 (JP)
(72) Inventor: KOHZUMA, Kaori, Sendai-shi, Miyagi 980-8577 (JP); MIYAMOTO, Koichiro, Sendai-shi, Miyagi 980-8577 (JP)
(74) Representative: Plasseraud IP
(86) International application number: PCT/JP2023/024792
(87) International publication number: WO 2024/010002

(57) **Abstract**

Disposed is a support structure for arranging an acquisition unit, which acquires information on a state of a leaf of a plant or information on a surrounding environment, in the vicinity of the leaf, including: a support that supports the acquisition unit; and a fixing portion that fixes the support to the plant, wherein the support is fixed by the fixing portion in a state in which the support extends from a side of a back surface of the leaf to a branch or a stem of the plant such that the acquisition unit is disposed on a side of the back surface of the leaf rather than on a side of a front surface of the leaf.

## Description

### TECHNICAL FIELD

The present invention relates to a support structure and a system.

Priority is claimed on Japanese Patent Application No. 2022-109088, filed July 06, 2022, the content of which is incorporated herein by reference.

### BACKGROUND ART

Due to a technology for measuring and detecting a physiological change in a plant in real time, it is possible to perform efficient remote observation in crop cultivation management in an agricultural field, environmental measurement in a difficult-to-reach area such as a forest, and the like. For long-term observation of a leaf of a plant, it is important not to damage the leaf or inhibit a physiological response such as photosynthesis, and it is also important to maintain robustness of attaching capability of an observation device. For example, Patent Document 1 discloses a plant sensing device that is attached to a leaf of a plant to acquire a close-up image of a leaf front surface. This plant sensing device includes a light source part having a surface-emitting light source, a sensor part having an image sensor and a fiber optic plate (FOP) disposed on an imaging surface thereof, and a part fixing means for pressing both the parts against a front surface of the leaf of the plant in a state in which a light emitting surface of the light source of the light source part and a front surface of the FOP of the sensor part oppose each other with the leaf of the plant sandwiched therebetween, thereby fixing both the parts to the leaf of the plant.

### RELATED ART DOCUMENT

### PATENT DOCUMENT

Patent Document 1: Japanese Unexamined Patent Application, First Publication No. 2021-56146

### SUMMARY

### PROBLEMS TO BE SOLVED BY THE INVENTION

However, since both the parts are fixed to the leaf of the plant with the leaf of the plant sandwiched therebetween, a structure is also provided on a side of a front surface of the leaf. For this reason, there is a high possibility that the leaf will be damaged or that a physiological response such as photosynthesis will be inhibited in long-term observation of the leaf of the plant.

The present invention has been made to solve the above-mentioned problems, and an object of the present invention is to provide a support structure and a system by which damage to a leaf of a plant or inhibition of photosynthesis during long-term observation of the leaf can be suppressed and robustness of attaching capability can be maintained.

### MEANS FOR SOLVING THE PROBLEM

As a means for solving the above problems, the aspects of the present invention have the following configurations.
(1) According to an aspect of the present invention, there is provided a support structure for arranging an acquisition unit, which acquires information on a state of a leaf of a plant or information on a surrounding environment, in the vicinity of the leaf, including: a support that supports the acquisition unit; and a fixing portion that fixes the support to the plant, wherein the support is fixed by the fixing portion in a state in which the support extends from a side of a back surface of the leaf to a branch or a stem of the plant such that the acquisition unit is disposed on a side of the back surface of the leaf rather than on a side of a front surface of the leaf.
   According to this configuration, since the acquisition unit is disposed on a side of the back surface of the leaf rather than on a side of the front surface of the leaf, there is no need to provide a structure on a side of the front surface of the leaf. For this reason, damage to the leaf of the plant or inhibition of photosynthesis during long-term observation of the leaf can be suppressed. In addition, since the support that supports the acquisition unit is fixed by the fixing portion in a state in which the support extends from a side of the back surface of the leaf to the branch or the stem of the plant, robustness of attaching capability can be maintained. Therefore, it is possible to provide the support structure by which the damage to the leaf of the plant or the inhibition of the photosynthesis during the long-term observation of the leaf can be suppressed and the robustness of the attaching capability can be maintained.
(2) In the support structure according to (1) above, the support may have elasticity.
(3) In the support structure according to (1) or (2) above, a plurality of acquisition units may be provided, and the plurality of acquisition units may be fixed to the support at different positions in a location at which the support overlaps the leaf.
(4) In the support structure according to any one of (1) to (3) above, the support may extend from a location at which the support overlaps the leaf to a location at which the support does not overlap the leaf, a plurality of acquisition units may be provided, and the plurality of acquisition units may include a leaf side acquisition unit that is fixed to the support in a location at which the support overlaps the leaf, and an outer side acquisition unit that is fixed to the support in a location at which the support does not overlap the leaf.
(5) In the support structure according to any one of (1) to (4) above, a plurality of fixing portions may be provided, and the plurality of fixing portions may include a leaf side fixing portion that fixes a portion of the support which is disposed on a side of the back surface of the leaf to the back surface of the leaf, and a branch-and-stem side fixing portion that fixes a portion of the support which is disposed on a side of the branch or the stem to the branch or the stem.
(6) The support structure according to any one of (1) to (5) above may further include a barrier that is fixed to the support and surrounds the acquisition unit to provide a space between the support and the back surface of the leaf.
(7) The support structure according to (6) above may further include a wall side fixing portion that fixes at least a part of a portion of the barrier on a side opposite to the support to the back surface of the leaf.
(8) In the support structure according to (7) above, an area of the wall side fixing portion may be smaller than an area of the entire portion of the barrier on a side opposite to the support.
(9) The support structure according to (7) or (8) above may further include an interposition portion that is interposed to fill a gap between a portion of the barrier on a side opposite to the support and the back surface of the leaf.
(10) In the support structure according to (9) above, the interposition portion may have a light blocking property.
(11) In the support structure according to any one of (1) to (10) above, the support may extend along a main vein on a side of the back surface of the leaf and then extend toward the branch or the stem of the plant.
(12) In the support structure according to any one of (1) to (11) above, an area of a portion of the support which is disposed on a side of the back surface of the leaf and is fixed to the back surface of the leaf may be smaller than an area of the entire back surface of the leaf.
(13) According to another aspect of the present invention, there is provided a system including: the support structure according to any one of (1) to (12) above; a light source that is supported on a portion of the support which is disposed on a side of the back surface of the leaf; a leaf side housing that accommodates a portion of the support which is disposed on a side of the back surface of the leaf together with the acquisition unit and the light source; a control device that controls the acquisition unit and the light source; and a branch-and-stem side housing that accommodates a portion of the support which is disposed on a side of the branch or the stem together with the control device and is fixed to the branch or the stem.
(14) The system according to (13) above may further include a power source that is accommodated in the branch-and-stem side housing and supplies power to the acquisition unit, the light source, and the control device.

### ADVANTAGE OF THE INVENTION

According to the present invention, it is possible to provide a support structure and a system by which damage to a leaf of a plant or inhibition of photosynthesis during long-term observation of the leaf can be suppressed and robustness of attaching capability can be maintained.

### BRIEF DESCRIPTION OF DRAWINGS

[FIG. 1] A perspective view showing an example in which a support structure of an embodiment is attached to a plant.
[FIG. 2] A perspective view showing a configuration example of the support structure of the embodiment.
[FIG. 3] A diagram showing an example in which the support structure of the embodiment is fixed to a back surface of a leaf.
[FIG. 4] A perspective view showing an example of a system that includes a part of the support structure of the embodiment.
[FIG. 5] An exploded perspective view of a leaf side housing in the system of the embodiment.
[FIG. 6] An exploded perspective view of a branch-and-stem side housing in the system of the embodiment.
[FIG. 7] A diagram showing an example of the arrangement of an acquisition unit and a light source in the system of the embodiment.
[FIG. 8] An explanatory diagram of step 1 of acquiring a reflection spectrum of a leaf using the acquisition unit and the light source in the system of the embodiment.
[FIG. 9] An explanatory diagram of step 2 of acquiring the reflection spectrum of the leaf using the acquisition unit and the light source in the system of the embodiment.
[FIG. 10] A perspective view showing an example in which the system of the embodiment is attached to a plant.
[FIG. 11] A perspective view showing an example in which a system of an example is attached to a plant.
[FIG. 12] A graph showing measurement data obtained by the system of the example, in which the upper part of FIG. 12 shows reflection spectrum data, and the lower part of FIG. 12 shows ambient light.

### DESCRIPTION OF EMBODIMENTS

Hereinafter, an embodiment of the present invention will be described with reference to the drawings. In the following embodiment, a structure for nondestructively measuring a physiological change in a plant in real time will be described as a support structure. In the drawings used in the following description, the scale of each member has been appropriately changed such that each member is of a recognizable size.

### <Support structure>

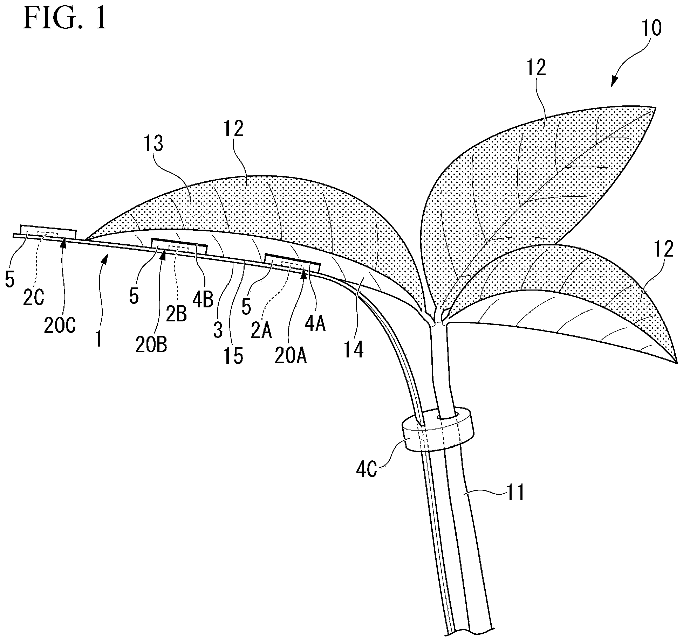

FIG. 1 is a perspective view showing an example in which a support structure 1 of an embodiment is attached to a plant 10. For example, the plant 10 is a terrestrial plant (a land plant) that grows on land. The plant 10 is not limited to the terrestrial plant. For example, the plant 10 may be an aquatic plant that grows underwater or on the surface of water. For example, the support structure 1 can be applied to any plant.

The plant 10 has a branch 11 (for example, a stalk) branching off from a stem and a plurality of leaves 12 provided on the branch 11. In the example shown in the figure, the branch 11 has three leaves 12 at a tip end thereof. In the example shown in the figure, the support structure 1 is provided along one leaf 12 of the three leaves 12 (for example, the largest leaf) and one branch 11.

The support structure 1 is a structure for arranging acquisition units 2A, 2B, and 2C, which acquire information on the state of the leaf 12 of the plant 10 or information on the surrounding environment, in the vicinity of the leaf 12. The support structure 1 includes a support 3 that supports the acquisition units 2A, 2B, and 2C, fixing portions 4A, 4B, and 4C that fix the support 3 to the plant 10, and a barrier 5 that surrounds each of the acquisition units 2A, 2B, and 2C to provide a space between the support 3 and a back surface 14 of the leaf. The support 3 is fixed by the fixing portions 4A, 4B, and 4C in a state in which the support 3 extends from a side of the back surface 14 of the leaf to the branch 11 of the plant 10 such that the acquisition units 2A, 2B, and 2C are disposed on a side of the back surface 14 of the leaf (corresponding to an abaxial side of the leaf) rather than on a side of a front surface 13 of the leaf (corresponding to an adaxial side of the leaf). In FIG. 1, the front surface 13 of the leaf is shown by dot hatching.

For example, each of the acquisition units 2A, 2B, and 2C is an optical sensor capable of optical measurement such as spectroscopic measurement. For example, the optical sensor may be a ready-made product such as S11059-02DT (manufactured by Hamamatsu Photonics K.K.). Each of the acquisition units 2A, 2B, and 2C is not limited to the optical sensor. For example, the acquisition units 2A, 2B, and 2C may be capable of measuring a temperature, a humidity, and the like in addition to the optical measurement. For example, each of the acquisition units 2A, 2B, and 2C may be a temperature sensor, a humidity sensor, a gas sensor, an ion sensor, an illuminance sensor, a vibration sensor, or the like. For example, the acquisition units 2A, 2B, and 2C may be capable of measuring physical and chemical evaluations of the leaf. For example, the acquisition units 2A, 2B, and 2C may be capable of measuring meteorological data in the vicinity of the leaf. For example, the acquisition units 2A, 2B, and 2C may be installed at any portions in the vicinity of the leaf. For example, detection of the environment may be realized by a color reaction with a pH test paper instead of the sensor or these may be combined. For example, the aspect of each of the acquisition units 2A, 2B, and 2C can be changed according to the required specifications.

For example, there may be a plurality of sensors within one barrier. For example, one acquisition unit may be capable of acquiring a plurality of pieces of information. In this case, a unit made up of the plurality of sensors may be placed inside one barrier, and thus the unit may function as an acquisition unit capable of acquiring the plurality of pieces of information. For example, the aspect in which the acquisition unit is installed in the barrier can be changed according to the required specifications.

The acquisition units 2A, 2B, and 2C are fixed to the support 3. A plurality of acquisition units 2A, 2B, and 2C are provided. In the example shown in the figure, three acquisition units 2A, 2B, and 2C are provided. The three acquisition units 2A, 2B, and 2C are spaced apart from each other in a direction in which the support 3 extends. The two acquisition units 2A and 2B are fixed to the support 3 at different positions in a location at which the support 3 overlaps the leaf 12. Here, a location at which the support 3 overlaps the leaf 12 means a location inside the outer periphery edge of the leaf 12 when viewed in a thickness direction of the leaf 12.

The support 3 extends from the location at which the support 3 overlaps the leaf 12 to a location at which the support 3 does not overlap the leaf 12. Here, a location at which the support 3 does not overlaps the leaf 12 means a location outside the outer periphery edge of the leaf 12 when viewed in a thickness direction of the leaf 12. The three acquisition units 2A, 2B, and 2C include leaf side acquisition units 2A and 2B fixed to the support 3 in a location at which the support 3 overlaps the leaf 12 and an outer side acquisition unit 2C fixed to the support 3 in a location at which the support 3 does not overlap the leaf 12. In the example shown in the figure, two leaf side acquisition units 2A and 2B and one outer side acquisition unit 2C are provided.

The support 3 extends along a main vein 15 on a side of the back surface 14 of the leaf and then extends toward the branch 11 of the plant 10. Here, the main vein 15 means the thickest leaf vein that extends through a central portion of the leaf from a tip end of the leaf toward a base of the leaf. In the example shown in the figure, the support 3 extends from a portion outside the tip end of the leaf 12 to a portion just before the base of the leaf 12 along the main vein 15 on a side of the back surface 14 of the leaf, then curves toward the branch 11 of the plant 10, and then extends along the branch 11.

The support 3 of the embodiment has elasticity. For example, the support 3 has a rectangular shape and uses a polyimide film or a liquid crystal polymer as a base film. For example, the support 3 is a flexible printed circuit board (hereinafter also simply referred to as a "flexible board") in which an electric circuit is formed on a substrate in which a thin insulating and flexible base film and a conductive metal are bonded together. For example, the width of the support 3 (the length in a direction perpendicular to a longitudinal direction and a thickness direction) may be 5 mm or more and 10 mm or less (for example, 7 mm). For example, the sensors that are the acquisition units 2A, 2B, and 2C may be capable of conducting electricity through the flexible board that is the support 3.

The support 3 is not limited to the flexible board. For example, the support 3 may be an elastic member such as a wire or a coil. For example, it is desirable that the support 3 be small and lightweight not to apply a load on the plant 10, but there are no limitations on the shape or material thereof. For example, it is desirable that the support 3 be able to support the support 3 itself and the barrier 5 by its elasticity, but there are no limitations on the shape or material thereof. For example, the aspect of the support 3 can be changed according to the required specifications.

A plurality of fixing portions 4A, 4B, and 4C are provided. The plurality of fixing portions 4A, 4B, and 4C include leaf side fixing portions 4A and 4B that fix a portion of the support 3 that is disposed on a side of the back surface 14 of the leaf to the back surface 14 of the leaf and a branch side fixing portion 4C (an example of a branch-and-stem side fixing portion) that fixes a portion of the support 3 that is disposed on a side of the branch 11 to the branch 11. Each of the leaf side fixing portions 4A and 4B is an example of a wall side fixing portion that fixes at least a part of a portion of the barrier 5 on a side opposite to the support 3 to the back surface 14 of the leaf.

For example, the leaf side fixing portions 4A and 4B are made of an adhesive such as a silicone-based adhesive or an acrylic-based adhesive. The method of fixing using the leaf side fixing portions 4A and 4B is not limited to that described above. For example, the material for fixing a portion of the barrier 5 on a side opposite to the support 3 to the back surface 14 of the leaf may be changed in accordance with the surface shape of the barrier 5. For example, it is desirable that at least one of the barriers 5 be pressed against the back surface 14 of the leaf with the elasticity of the support 3. For example, the aspect of each of the leaf side fixing portions 4A and 4B can be changed according to the required specifications.

For example, the branch side fixing portion 4C is a string-like member. For example, a portion of the support 3 that is disposed on a side of the branch 11 is fixed by being tied to a part of the branch 11 using a string-like member that is the branch side fixing portion 4C. The method of fixing using the branch side fixing portion 4C is not limited to that described above. For example, fixation using the branch side fixing portion 4C may be by adhesion or suction. For example, the branch side fixing portion 4C may be a clip or clamp that clamps and fixes a part of the branch 11 and the support 3 together. For example, the size of the branch side fixing portion 4C is not limited. For example, the number of branch side fixing portions 4C may be multiple. For example, the branch side fixing portion 4C does not need to be fixed to the branch 11 in a state in which the support 3 itself is in contact with the branch 11, and may be fixed to the branch 11 via a connector or connector board connected to the support 3. For example, the aspect of each of the branch side fixing portion 4C can be changed according to the required specifications.

The barrier 5 is fixed to the support 3. Three barriers 5 are provided corresponding to the three acquisition units 2A, 2B, and 2C. The three barriers 5 are spaced apart from each other in a direction in which the support 3 extends. Each of the barriers 5 is provided to surround one of the acquisition units 2A, 2B, and 2C.

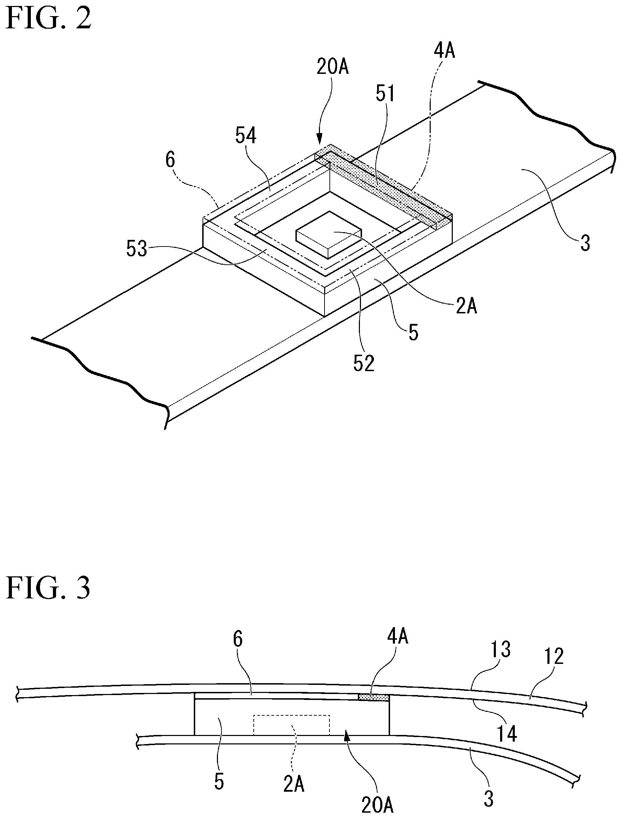

FIG. 2 is a perspective view showing a configuration example of the support structure 1 of the embodiment. FIG. 3 is a diagram showing an example in which the support structure 1 of the embodiment is fixed to the back surface 14 of the leaf. In the example shown in the figure, the periphery of the barrier 5 surrounding the leaf side acquisition unit 2A is shown. The barrier 5 is formed in a rectangular frame shape. For example, the barrier 5 may have a light blocking property. For example, the barrier 5 may be formed of a black plastic material. The barrier 5 is provided to reduce disturbances during measurement, and the material thereof is not limited thereto. For example, the aspect of the barrier 5 is selected according to the acquisition units 2A, 2B, and 2C and the type of measurement. For example, the barrier 5 may be formed of an opaque material to block ambient light. For example, the barrier 5 may be formed of an impermeable material to block the inflow of gas. For example, the aspect of the barrier 5 can be changed according to the required specifications. For example, an opening portion may be provided in a part of the barrier 5. For example, gas exchange may occur through the opening portion of the barrier 5.

For example, the support 3 may be provided with a light source for optical measurement together with small spectroscopic sensors which are the acquisition units 2A, 2B, and 2C. For example, the light source may be an LED. For example, the support 3 may be provided with a gas generation source for gas measurement. For example, the barrier 5 may be provided to surround the periphery of the small spectroscopic sensor, the light source, and the like. In the example of FIG. 1, the height of the barrier 5 is greater than the thickness of an element of the acquisition unit (for example, a sensor). For example, the barrier 5 may function as a spacer if necessary to maintain an appropriate distance between a sensor upper surface and the back surface 14 of the leaf for measurement. For example, the size (particularly the height) of the barrier 5 can be changed according to a required specification.

Hereinafter, a structure including the small spectroscopic sensor (the acquisition unit 2A, 2B, or 2C), the light source, and the like, and the barrier 5 is also referred to as a "chamber 20A, 20B, or 20C." In the example of FIG. 1, three (an example of multiple) chambers 20A, 20B, and 20C are provided. Although the barrier 5 is formed in a rectangular frame shape, the shape is not limited thereto. For example, the barrier 5 may be provided to surround the periphery of the sensor in accordance with the shape and arrangement of the sensor. For example, each of the chambers 20A, 20B, and 20C may include a light source for optical measurement, a gas generation source for gas measurement, an air replacement mechanism, an air stirring mechanism, a chemical substance generation mechanism, and the like, in addition to the sensor. For example, each of the chambers 20A, 20B, and 20C may include elements and mechanisms necessary for constructing a measurement system. For example, the configuration aspect of each of the chambers 20A, 20B, and 20C can be changed according to the required specifications.

For example, in a case in which each of the acquisition units 2A, 2B, and 2C such as sensors is of a type that does not require the barrier 5, each of the chambers 20A, 20B, and 20C may be constituted by only the sensor and the like. In this case, the sensor and the like may be fixed directly to the back surface 14 of the leaf. Alternatively, the support 3 around the sensor and the like may be deformed, and thus the support 3 may be fixed directly to the back surface 14 of the leaf. For example, one or more of the chambers 20A, 20B, and 20C may be installed on the back surface 14 of the leaf without adhering thereto, according to the measurement requirements. In this case, a part of the support 3 may be fixed to the back surface 14 of the leaf. For example, the aspect in which the constituent elements of the support structure 1 are fixed to the back surface 14 of the leaf can be changed according to the required specifications.

In the present embodiment, the small spectroscopic sensor and the light source are mounted on a flexible board having a width of 7 mm as the support 3, and a black plastic barrier 5 is disposed around them to form each of the chambers 20A, 20B, and 20C. The color and material of the barrier 5 are not limited to those described above and can be changed according to a required specification. For example, communication with the sensor and control of the light source may be performed by a microcontroller or any personal computer (an example of a control device). For example, by utilizing the communication function of the control device, it is possible to acquire measurement data remotely.

In the present embodiment, the area of each of the leaf side fixing portions 4A and 4B (an example of a wall side fixing portion) is smaller than the area of the entire portion of the barrier 5 on a side opposite to the support 3. Here, the area of the entire portion of the barrier 5 on a side opposite the support 3 means the area of the entire portion of the barrier 5 on a side of the back surface 14 of the leaf. The area of the entire portion of the barrier 5 on a side opposite the support 3 corresponds to the area of the rectangular frame-shaped barrier 5 on a side of the back surface 14 of the leaf along four sides of the rectangular frame-shaped barrier 5. In the example of FIG. 2, the leaf side fixing portion 4A is disposed on a surface that is along only one side 51 of four sides 51, 52, 53, and 54 of the rectangular frame-shaped barrier 5. In this case, the area of the leaf side fixing portion 4A is about one quarter of the area of the entire portion of the barrier 5 on a side opposite the support 3.

The support structure 1 of the present embodiment further includes an interposition portion 6 that is interposed to fill a gap between a portion of the barrier 5 on a side opposite to the support 3 and the back surface 14 of the leaf. In the example of FIG. 2, the interposition portion 6 is disposed on a surface that is along only three sides 52, 53, and 54 of the four sides 51, 52, 53, and 54 of the rectangular frame-shaped barrier 5. The interposition portion 6 is formed along the sides 52, 53, and 54 of the four sides 51, 52, 53, and 54 of the rectangular frame-shaped barrier 5 on which the leaf side fixing portion 4A is not disposed. The interposition portion 6 is formed in a U shape along the sides 52, 53, and 54.

In the present embodiment, the interposition portion 6 has a light blocking property. For example, the interposition portion 6 is formed of a black material such as rubber or sponge. The interposition portion 6 is provided to reduce a pressing force occurring when the barrier 5 is pressed against the back surface 14 of the leaf, and the material thereof is not limited thereto. For example, the aspect of the interposition portion 6 is selected according to the magnitude of the pressing force. For example, the interposition portion 6 may be formed of an opaque material to block ambient light. For example, the interposition portion 6 may be formed of an impermeable material to block the inflow of gas. For example, the aspect of the interposition portion 6 can be changed according to the required specifications.

In the present embodiment, the area of a portion of the support 3 which is disposed on a side of the back surface 14 of the leaf and is fixed to the back surface 14 of the leaf is smaller than the area of the entire back surface 14 of the leaf. In the example shown in the figure, the area of a portion of the support 3 which is disposed on a side of the back surface 14 of the leaf and is fixed to the back surface 14 of the leaf is the area of the leaf side fixing portions 4A and 4B.

### <Operations and effects>

As described above, the support structure 1 according to the present embodiment is a structure for arranging the acquisition units 2A, 2B, and 2C, which acquire information on the state of the leaf 12 of the plant 10 or information on the surrounding environment, in the vicinity of the leaf 12. The support structure 1 includes the support 3 that supports the acquisition units 2A, 2B, and 2C and the fixing portions 4A, 4B, and 4C that fix the support 3 to the plant 10. The support 3 is fixed by the fixing portions 4A, 4B, and 4C in a state in which the support 3 extends from a side of the back surface 14 of the leaf to the branch 11 of the plant 10 such that the acquisition units 2A, 2B, and 2C are disposed on a side of the back surface 14 of the leaf rather than on a side of the front surface 13 of the leaf.

According to this configuration, since the acquisition units 2A, 2B, and 2C are disposed on a side of the back surface 14 of the leaf rather than on a side of the front surface 13 of the leaf, there is no need to provide a structure on a side of the front surface 13 of the leaf. For this reason, damage to the leaf of the plant or inhibition of photosynthesis during long-term observation of the leaf can be suppressed. In addition, since the support 3 that supports the acquisition units 2A, 2B, and 2C is fixed by the fixing portions 4A, 4B, and 4C in a state in which the support 3 extends from a side of the back surface 14 of the leaf to the branch 11 of the plant 10, robustness of attaching capability can be maintained. Therefore, it is possible to provide the support structure 1 by which the damage to the leaf of the plant or the inhibition of the photosynthesis during the long-term observation of the leaf can be suppressed and the robustness of the attaching capability can be maintained.

The support 3 according to the present embodiment has elasticity.

According to this configuration, it is possible to press the acquisition units 2A, 2B, and 2C against the back surface 14 of the leaf with the elasticity of the support 3.

According to the present embodiment, the plurality of acquisition units 2A and 2B are provided. The plurality of acquisition units 2A and 2B are fixed to the support 3 at different positions in a location at which the support 3 overlaps the leaf 12.

According to this configuration, information on the state of different locations on the back surface 14 of the leaf or information on the surrounding environment can be acquired by the plurality of acquisition units 2A and 2B.

The support 3 according to the present embodiment extends from the location at which the support 3 overlaps the leaf 12 to the location at which the support 3 does not overlap the leaf 12. The plurality of acquisition units 2A, 2B, and 2C are provided. The plurality of acquisition units 2A, 2B, and 2C include the leaf side acquisition units 2A and 2B fixed to the support 3 in a location at which the support 3 overlaps the leaf 12 and the outer side acquisition unit 2C fixed to the support 3 in a location at which the support 3 does not overlap the leaf 12.

According to this configuration, it is possible to acquire information on the location at which the support 3 overlaps the leaf 12 by the leaf side acquisition units 2A and 2B, and it is possible to acquire information on the location at which the support 3 does not overlap the leaf 12 by the outer side acquisition unit 2C. For example, in a case in which each of the leaf side acquisition units 2A and 2B is the optical sensor, it is possible to measure light that has been transmitted from a side of the front surface 13 to a side of the back surface 14 of the leaf (transmitted light). For example, in a case in which the outer side acquisition unit 2C is the optical sensor, it is possible to measure light that is not the transmitted light (for example, ambient light). For example, data regarding the amount of light absorption by the leaf 12 can be acquired on the basis of the transmitted light and the ambient light.

According to the present embodiment, the plurality of fixing portions 4A, 4B, and 4C are provided. The plurality of fixing portions 4A, 4B, and 4C include the leaf side fixing portions 4A and 4B that fix a portion of the support 3 that is disposed on a side of the back surface 14 of the leaf to the back surface 14 of the leaf and the branch side fixing portion 4C that fixes a portion of the support 3 that is disposed on a side of the branch 11 to the branch 11.

According to this configuration, the weight of the support 3 is dispersed to the leaf side fixing portions 4A and 4B and the branch side fixing portion 4C, and thus it is possible to prevent the plant 10 from being damaged.

The support structure 1 according to the present embodiment further includes the barrier 5 that is fixed to the support 3 and surrounds each of the acquisition units 2A, 2B, and 2C to provide a space between the support 3 and the back surface 14 of the leaf.

According to this configuration, each of the acquisition units 2A, 2B, and 2C is surrounded by the barrier 5, and thus the acquisition units 2A, 2B, and 2C can be protected from external factors. In the present embodiment, the barrier 5 is supported with the elasticity of the support 3, and thus it is possible to prevent excessive load from being applied to a contact surface between the back surface 14 of the leaf and the barrier 5. In addition, even in a case in which a contact area between the back surface 14 of the leaf and the barrier 5 is small, the acquisition units 2A, 2B, and 2C can be installed stably for a long period of time. Furthermore, since the contact area between the back surface 14 of the leaf and the barrier 5 is small, the growth and deformation of the leaf 12 is not hindered. In addition, the barrier 5 is pressed against the back surface 14 of the leaf, and thus it is possible for the barrier 5 to reduce a disturbance.

The support structure 1 according to the present embodiment further includes the wall side fixing portions 4A and 4B (the leaf side fixing portions), each of which fixes at least a part of a portion of the barrier 5 on a side opposite to the support 3 to the back surface 14 of the leaf.

According to this configuration, it is possible to fix a portion of the barrier 5 on a side opposite to the support 3 to the back surface 14 of the leaf by each of the wall side fixing portions 4A and 4B. For example, it is easy to maintain the robustness of the attaching capability compared to a case in which the acquisition units 2A and 2B themselves are fixed to the back surface 14 of the leaf or a case in which the support 3 itself is fixed to the back surface 14 of the leaf. In the present embodiment, the plurality of barriers 5 are fixed to the back surface 14 of the leaf by corresponding wall side fixing portions 4A and 4B. As a result, it is possible to disperse the load applied on the leaf 12, and thus it is easy to prevent the leaf 12 from being damaged compared to a case in which only one barrier 5 is fixed to the back surface 14 of the leaf.

The area of each of the wall side fixing portions 4A and 4B according to the present embodiment is smaller than the area of the entire portion of the barrier 5 on a side opposite to the support 3.

According to this configuration, it is possible to make it less likely that the growth and deformation of the leaf 12 is hindered compared to a case in which the entire portion of the barrier 5 on a side opposite to the support 3 is fixed to the back surface 14 of the leaf.

The support structure 1 according to the present embodiment further includes the interposition portion 6 that is interposed to fill a gap between a portion of the barrier 5 on a side opposite to the support 3 and the back surface 14 of the leaf.

According to this configuration, a gap between a portion of the barrier 5 on a side opposite to the support 3 and the back surface 14 of the leaf is filled with the interposition portion 6, and thus the acquisition units 2A and 2B can be protected from external factors.

The interposition portion 6 according to the present embodiment has a light blocking property.

According to this configuration, the interposition portion 6 can block light (for example, the ambient light) traveling toward each of the acquisition units 2A and 2B from the gap between a portion of the barrier 5 on a side opposite to the support 3 and the back surface 14 of the leaf. For example, in a case in which each of the acquisition units 2A and 2B is the optical sensor, it is possible to measure light that has been transmitted from a side of the front surface 13 to a side of the back surface 14 of the leaf (transmitted light) with high accuracy without being affected by the ambient light and the like.

The support 3 according to the present embodiment extends along the main vein 15 on a side of the back surface 14 of the leaf and then extends toward the branch 11 of the plant 10.

According to this configuration, it is possible to maintain the robustness of the attaching capability compared to a case in which the support 3 extends across the main vein 15 on a side of the back surface 14 of the leaf.

According to the present embodiment, the area of a portion of the support 3 which is disposed on a side of the back surface 14 of the leaf and is fixed to the back surface 14 of the leaf is smaller than the area of the entire back surface 14 of the leaf.

According to this configuration, it is possible to make it less likely that the growth and deformation of the leaf 12 is hindered compared to a case in which the entire portion of the support 3 which is disposed on a side of the back surface 14 of the leaf is fixed to the back surface 14 of the leaf.

The technical scope of the present invention is not limited to the above-described embodiments, and various modifications can be made without departing from the spirit of the present invention.

In the above-described embodiment, an example in which the support is fixed to the branch of the plant in a state in which the support extends from a side of the back surface of the leaf to the branch such that the acquisition units are disposed on the back surface of the leaf rather than on a side of the front surface of the leaf has been described, but the present invention is not limited to this. For example, the support may be fixed to the stem in a state in which the support extends from a side of the back surface of the leaf to the stem of the plant such that the acquisition units are disposed on a side of the back surface of the leaf rather than on a side of the front surface of the leaf. For example, the aspect in which the support extends from a side of the back surface of the leaf to the branch or the stem can be changed according to the required specifications.

In the above-described embodiment, an example in which the support has elasticity has been described, but the present invention is not limited to this. For example, the support may not have elasticity. For example, the support may be a member that can be considered as a rigid body. For example, the aspect of the support can be changed according to the required specifications.

In the above-described embodiment, an example in which the plurality of acquisition units are fixed to the support at different positions in a location at which the support overlaps the leaf has been described, but the present invention is not limited to this. For example, only one acquisition unit may be fixed to the back surface of the leaf. For example, the installation aspect of the acquisition unit can be changed according to the required specifications.

In the above-described embodiment, an example in which the plurality of acquisition units include the leaf side acquisition units fixed to the support in a location at which the support overlaps the leaf and the outer side acquisition unit fixed to the support in a location at which the support does not overlap the leaf has been described, but the present invention is not limited to this. For example, the acquisition unit may not be provided in a location at which the support does not overlap the leaf. For example, the acquisition unit may be provided only in a location at which the support overlaps the leaf. For example, the acquisition unit may be provided only in a location at which the support does not overlap the leaf. For example, the installation aspect of the acquisition unit can be changed according to the required specifications.

In the above-described embodiment, an example in which the plurality of fixing portions include the leaf side fixing portions that fix a portion of the support that is disposed on a side of the back surface of the leaf to the back surface of the leaf and the branch side fixing portion that fixes a portion of the support that is disposed on a side of the branch to the branch has been described, but the present invention is not limited to this. For example, a portion of the support which is disposed on a side of the back surface of the leaf may not be fixed to the back surface of the leaf. For example, only a portion of the support which is disposed on a side of the branch or the stem may be fixed to the branch or the stem. For example, the fixation aspect of the support can be changed according to the required specifications.

**In** the above-described embodiment, an example in which the support structure includes the barrier that is fixed to the support and surrounds each of the acquisition units to provide a space between the support and the back surface of the leaf has been described, but the present invention is not limited to this. For example, the support structure may not include the barrier. For example, the acquisition unit may be surrounded by the support. For example, the acquisition unit may be exposed to an external space. For example, the aspect in which the acquisition unit is protected can be changed according to the required specifications.

In the above-described embodiment, an example in which the support structure includes the wall side fixing portions, each of which fixes at least a part of a portion of the barrier on a side opposite to the support to the back surface of the leaf has been described, but the present invention is not limited to this. For example, the support structure may not include the wall side fixing portions. For example, the acquisition unit itself may be fixed to the back surface of the leaf. For example, the support itself may be fixed to the back surface of the leaf. For example, the fixation aspect of the barrier can be changed according to the required specifications.

In the above-described embodiment, an example in which the area of each of the wall side fixing portions is smaller than the area of the entire portion of the barrier on a side opposite to the support has been described, but the present invention is not limited to this. For example, the entire portion of the barrier on a side opposite to the support may be fixed to the back surface of the leaf. For example, the area of each of the wall side fixing portions may be the same as the area of the entire portion of the barrier on a side opposite to the support. For example, the area of each of the wall side fixing portions can be changed according to a required specification.

In the above-described embodiment, an example in which the support structure includes the interposition portion that is interposed to fill a gap between a portion of the barrier on a side opposite to the support and the back surface of the leaf has been described, but the present invention is not limited to this. For example, the support structure may not include the interposition portion. For example, there may be a gap between a portion of the barrier on a side opposite to the support and the back surface of the leaf. For example, the installation aspect of the interposition portion can be changed according to the required specifications.

In the above-described embodiment, an example in which the interposition portion has a light blocking property has been described, but the present invention is not limited to this. For example, the interposition portion may not have a light blocking property. For example, the interposition portion may be a member having a light transmissive property. For example, the aspect of the interposition portion can be changed according to the required specifications.

In the above-described embodiment, an example in which the support extends along the main vein on a side of the back surface of the leaf and then extends toward the branch of the plant has been described, but the present invention is not limited to this. For example, the support may extend across the main vein on a side of the back surface of the leaf. For example, the aspect in which the support extends can be changed according to the required specifications.

In the above-described embodiment, an example in which the area of a portion of the support which is disposed on a side of the back surface of the leaf and is fixed to the back surface of the leaf is smaller than the area of the entire back surface of the leaf has been described, but the present invention is not limited to this. For example, the entire portion of the support which is disposed on a side of the back surface of the leaf may not be fixed to the back surface of the leaf. For example, the area of a portion of the support which is disposed on a side of the back surface of the leaf and is fixed to the back surface of the leaf may be the same as the area of the entire back surface of the leaf. For example, the area of a portion of the support which is disposed on a side of the back surface of the leaf and is fixed to the back surface of the leaf can be changed according to a required specification.

### <System>

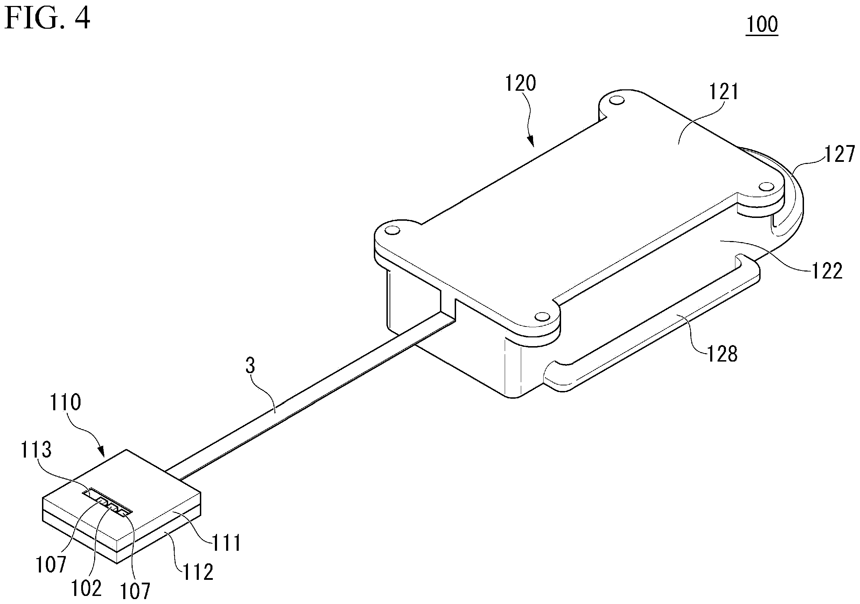

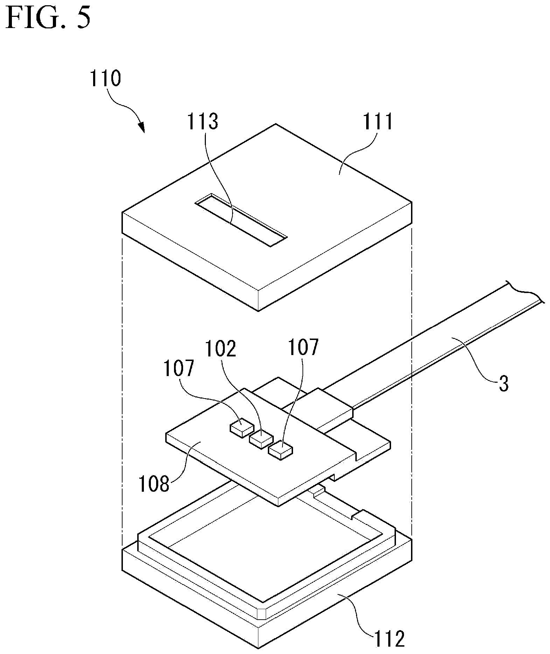

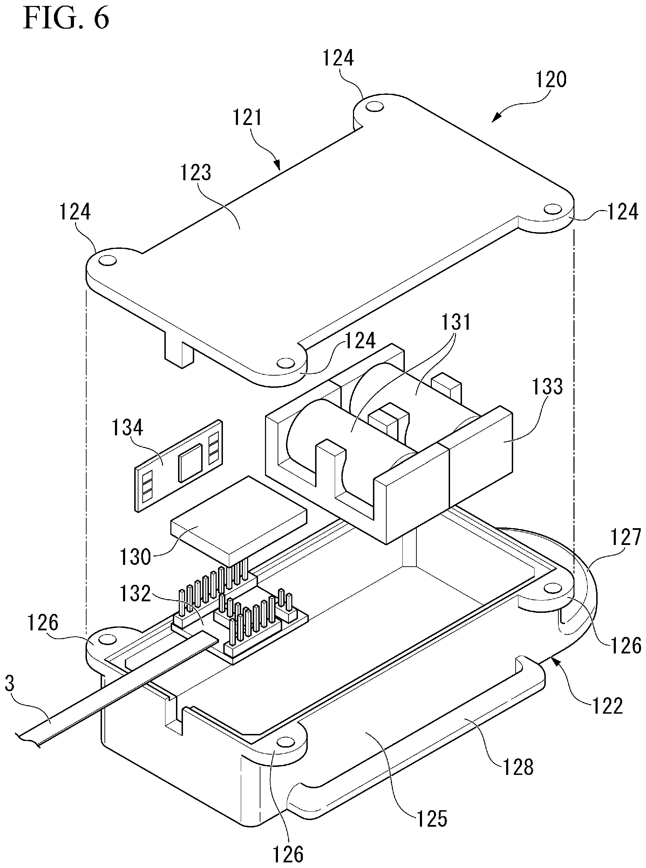

FIG. 4 is a perspective view showing an example of a system 100 that includes a part of the support structure of the embodiment. FIG. 5 is an exploded perspective view of a leaf side housing 110 in the system 100 of the embodiment. FIG. 6 is an exploded perspective view of a branch-and-stem side housing 120 in the system 100 of the embodiment. In the following description, the same constituent elements as those in the above-described embodiment are designated by the same reference signs, and the detailed description thereof will be omitted.

Referring also to FIGS. 4 to 6 together, some of the support structure 1 described above (the support 3 and the like in the example shown in the figure) may be applied to the measurement system 100 for outdoor use which is powered by a battery that is a power source 131.

The system 100 includes a part of the support structure 1 (an example of at least a part of the support structure), a light source 107 that is supported on a portion of the support 3 which is disposed on a side of the back surface 14 of the leaf 12, a leaf side housing 110 that accommodates a portion of the support 3 which is disposed on a side of the back surface 14 of the leaf 12 together with an acquisition unit 102 and the light source 107, a control device 130 that controls the acquisition unit 102 and the light source 107, a power source 131 that supplies power to the acquisition unit 102, the light source 107, and the control device 130, and a branch-and-stem side housing 120 that accommodates a portion of the support 3 which is disposed on a side of the branch 11 or the stem together with the control device 130 and the power source 131 and is fixed to the branch 11 or the stem. The system 100 is not limited to that including only a part of the support structure 1 described above and may include the entire support structure 1 described above. For example, the configuration aspect of the system 100 can be changed according to the design specifications.

In the example shown in the figure, one small spectroscopic sensor (hereinafter also simply referred to as a "sensor") serving as the acquisition unit 102 and two LEDs each serving as the light source 107 are provided. In the example shown in the figure, the two LEDs are arranged in a row with one sensor interposed therebetween. The number of acquisition units 102 and light sources 107 installed and their arrangement aspect are not limited to the above and can be changed according to the design specifications.

The acquisition unit 102 and the light source 107 are supported on a portion of the support 3 which is disposed on a side of the back surface 14 of the leaf 12 via a connector board 108. The acquisition unit 102 and the light source 107 may be supported directly on a portion of the support 3 which is disposed on a side of the back surface 14 of the leaf 12 without the connector board 108 being interposed therebetween. For example, the support aspect of each of the acquisition unit 102 and the light source 107 can be changed according to the design specifications.

The leaf side housing 110 accommodates a portion of the support 3 which is disposed on a side of the back surface 14 of the leaf 12 together with the acquisition unit 102, the light source 107, and the connector board 108. The leaf side housing 110 is constituted by an upper cover 111 that is disposed to cover a portion of the support 3 which is disposed on a side of the back surface 14 of the leaf 12 together with the acquisition unit 102, the light source 107, and the connector board 108 from above and a lower cover 112 that is disposed to cover the connector board 108 from below.

For example, the leaf side housing 110 may have a function of fixing the sensor to the back surface of the leaf while maintaining an appropriate distance between the sensor upper surface and the back surface 14 of the leaf, like the barrier 5 shown in FIG. 2. For example, the leaf side housing 110 may be painted black or formed of a black material to have a light blocking property. For example, the leaf side housing 110 may have at least one of the function of the support 3 and the function of the barrier 5. For example, the function and the configuration aspect of the leaf side housing 110 can be changed according to the design specifications.

The upper cover 111 has a rectangular slit 113 formed therein, which exposes the acquisition unit 102 and the light source 107 when viewed from above. The shape of the slit 113 is not limited to the above and may be elliptical when viewed from above. For example, the formation aspect of the slit 113 can be changed according to the design specifications.

The outer shape of the upper cover 111 is rectangular and larger than the outer shape of the connector board 108 which is rectangular when viewed from above. The outer shape of the upper cover 111 is not limited to the above and may be circular when viewed from above. For example, the shape of the upper cover 111 can be changed according to the design specifications.

The outer shape of the lower cover 112 is rectangular and larger than the outer shape of the connector board 108 which is rectangular when viewed from above. For example, the outer shape of the lower cover 112 is the same size as the outer shape of the upper cover 111 when viewed from above. The outer shape of the lower cover 112 is not limited to the above and may be circular when viewed from above. For example, the shape of the lower cover 112 can be changed according to the design specifications.

For example, a portion of the support 3 which is disposed on a side of the back surface 14 of the leaf 12 is accommodated in an internal space of the lower cover 112 together with the acquisition unit 102, the light source 107, and the connector board 108, and then the upper cover 111 is fitted to the lower cover 112 (an example of joining) to form the leaf side housing 110. For example, a joint portion between the upper cover 111 and the lower cover 112 may be sealed with a sealing material (not shown) such as silicone sealant. As a result, it is possible to maintain a waterproofing property sufficient to withstand the long-term outdoor measurement.

For example, the control device 130 is a microcontroller. The control device 130 controls one sensor serving as the acquisition unit 102 and two LEDs each serving as the light source 107. The control device 130 controls the sensor and the LEDs to acquire data regarding the amount of light absorption of the leaf 12, and the like. The control device 130 has not only a function of controlling the sensor and the like, but also a function of calculating data. The control device 130 has a wireless communication function in addition to a wired communication function. An example of the control device 130 includes a small and low-power microcontroller that has functions such as WiFi (a registered trademark) and Bluetooth (a registered trademark). For example, the control device 130 may transfer the acquired data to an external device (for example, a personal computer, a smartphone, an online storage, or the like) via wireless communication. For example, the aspect in which data is integrated can be changed according to the design specifications.

The control device 130 is supported via an expansion board 132 on a portion of the support 3 which is disposed on a side of the branch 11 or the stem. The expansion board 132 is an electronic board on which electronic components are mounted to add and enhance the functions of the control device 130, and connection terminals are arranged in a part of the expansion board. The control device 130 may be supported directly on a portion of the support 3 which is disposed on a side of the branch 11 or the stem without the expansion board 132 being interposed therebetween. For example, the support aspect of the control device 130 can be changed according to the design specifications.

For example, the power source 131 is a lithium battery. The power source 131 supplies power to each of the one sensor serving as the acquisition unit 102, the two LEDs each serving as the light source 107, and the control device 130. In the example shown in the figure, two power sources 131 are held by a holder 133. The power source 131 may be a battery other than a lithium battery. For example, one power source 131 or three or more power sources 131 may be provided. For example, the power source 131 does not have to be held by the holder 133. For example, the type of the power source 131, the number of power sources 131 installed, and the holding aspect of the power source 131 can be changed according to the design specifications.

The branch-and-stem side housing 120 accommodates a portion of the support 3 which is disposed on a side of the branch 11 or the stem together with the control device 130, the expansion board 132, the power source 131, the holder 133, and a voltage regulator 134. The voltage regulator 134 is an electronic component equipped with an integrated circuit that keeps the output voltage of the power source 131 constant according to the input voltage, the output current, and the load resistance.

For example, the aspect in which each component is accommodated within the housing (such as the structure of an accommodation portion) can be changed according to the design specifications. For example, in a case in which the support is accommodated, a spring type using a spring (not shown) can be adopted.

The branch-and-stem side housing 120 is constituted by: an upper case 121 disposed to cover from above a portion of the support 3 which is disposed on a side of the branch 11 or the stem together with the control device 130, the expansion board 132, the power source 131, the holder 133, and the voltage regulator 134; and a lower case 122 disposed to cover from below a portion of the support 3 which is disposed on a side of the branch 11 or the stem together with the control device 130, the expansion board 132, the power source 131, the holder 133, and voltage regulator 134.

The upper case 121 is constituted by an upper base 123 having a rectangular shape when viewed from above and an upper protrusion 124 having a through hole formed therein and protruding outward from each of four corners of the upper base 123. The outer shape of the upper base 123 is rectangular and larger than a region including a portion of the support 3 which is disposed on a side of the branch 11 or the stem, the control device 130, the expansion board 132, the power source 131, the holder 133, and the voltage regulator 134 when viewed from above. The shape of the upper base 123 is not limited to the above and can be changed according to the design specifications.

The lower case 122 is constituted by a cylindrical lower base 125 with a bottom having a rectangular shape when viewed from above, a lower protrusion 126 having a through hole formed therein and protruding outward from each of upper four corners of the lower base 125, a U-shaped first engagement portion 127 disposed on a side opposite to a portion of the lower base 125 through which the support 3 passes, and a C-shaped second engagement portion 128 disposed on a lower side portion of the lower base 125.

The outer shape of the lower base 125 is rectangular and larger than a region including a portion of the support 3 which is disposed on a side of the branch 11 or the stem, the control device 130, the expansion board 132, the power source 131, the holder 133, and the voltage regulator 134 when viewed from above. For example, the outer shape of the lower base 125 is the same size as the outer shape of the upper base 123 when viewed from above. The shape of the lower base 125 is not limited to the above and can be changed according to the design specifications.

For example, the control device 130, the expansion board 132, the power source 131, the holder 133, and the voltage regulator 134 are accommodated in the internal space of the lower case 122 together with a portion of the support 3 which is disposed on a side of the branch 11 or the stem, and then the upper case 121 can be fitted into the lower case 122 (an example of joining) to form the branch-and-stem side housing 120. For example, the joint portion between the upper case 121 and the lower case 122 may be sealed with a sealing material (not shown) such as a silicone sealant. At this time, since the upper protrusion 124 and the lower protrusion 126 protrude, a fitting portion becomes flat, making it easier to apply the sealing material uniformly. As a result, it is possible to maintain a waterproofing property sufficient to withstand the long-term outdoor measurement.

For example, in a case in which the joint portion between the upper case 121 and the lower case 122 is sealed with the sealing material, the upper protrusion 124 and the lower protrusion 126 may be temporarily secured (one example of fixing) with a screw or the like through each through hole. As a result, it is possible to stably fix the upper case 121 and the lower case 122 to each other. The aspect in which the upper case 121 and the lower case 122 are fixed to each other is not limited to the above and can be changed according to the design specifications.

### <Method for acquiring reflection spectrum of leaf>

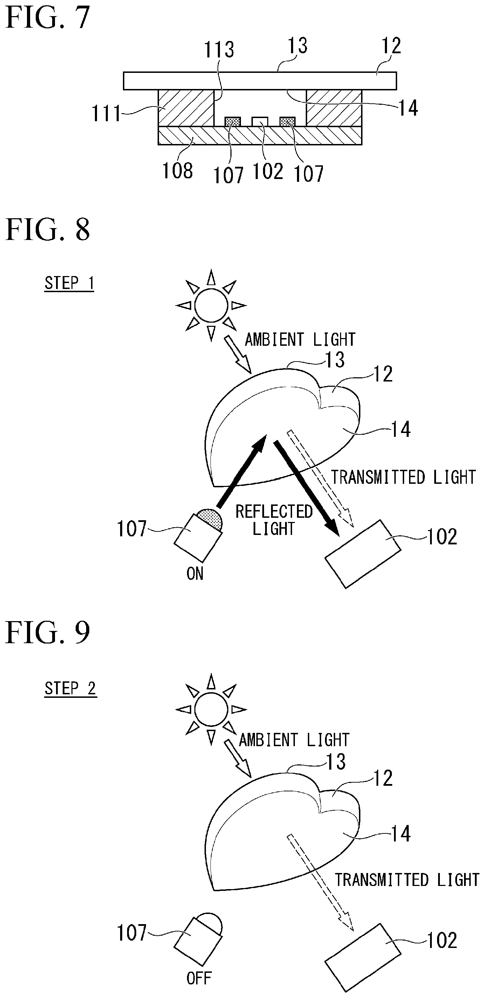

FIG. 7 is a diagram showing an example of the arrangement of the acquisition unit 102 and the light source 107 in the system 100 of the embodiment. FIG. 8 is an explanatory diagram of step 1 of acquiring a reflection spectrum of the leaf 12 using the acquisition unit 102 and the light source 107 in the system 100 of the embodiment. FIG. 9 is an explanatory diagram of step 2 of acquiring the reflection spectrum of the leaf 12 using the acquisition unit 102 and the light source 107 in the system 100 of the embodiment.

Referring to FIGS. 7 to 9 together, the control device 130 acquires the reflection spectrum of the leaf 12 using a sensor serving as the acquisition unit 102 and an LED serving as the light source 107.

The control device 130 acquires the reflection spectrum through step 1 and step 2. The ambient light, such as sunlight, is transmitted through the leaf 12. In step 1, the control device 130 acquires data from the sensor in a state in which the LED is turned on. In step 1, the control device 130 acquires the transmitted light (the light obtained by transmitting the ambient light from the front surface 13 to the back surface 14 of the leaf 12) and the reflected light (the light obtained by reflecting the LED light by the back surface 14 of the leaf 12). In step 2, the control device 130 acquires data from the sensor in a state in which the LED is turned off. In step 2, the control device 130 acquires only the transmitted light.

The control device 130 acquires the reflected light intensity from the back surface 14 of the leaf 12 by calculating the difference between the data obtained in step 1 and step 2. The control device 130 acquires the reflection spectrum on the basis of the acquired reflected light intensity and parameters such as a wavelength. The control device 130 may transfer the data after performing calculation processing, or may transfer all of the unprocessed data. The control device 130 can acquire the intensity of the ambient light and the intensity of light correlated with the ambient light using only the data from step 2.

### <Method for fixing system>

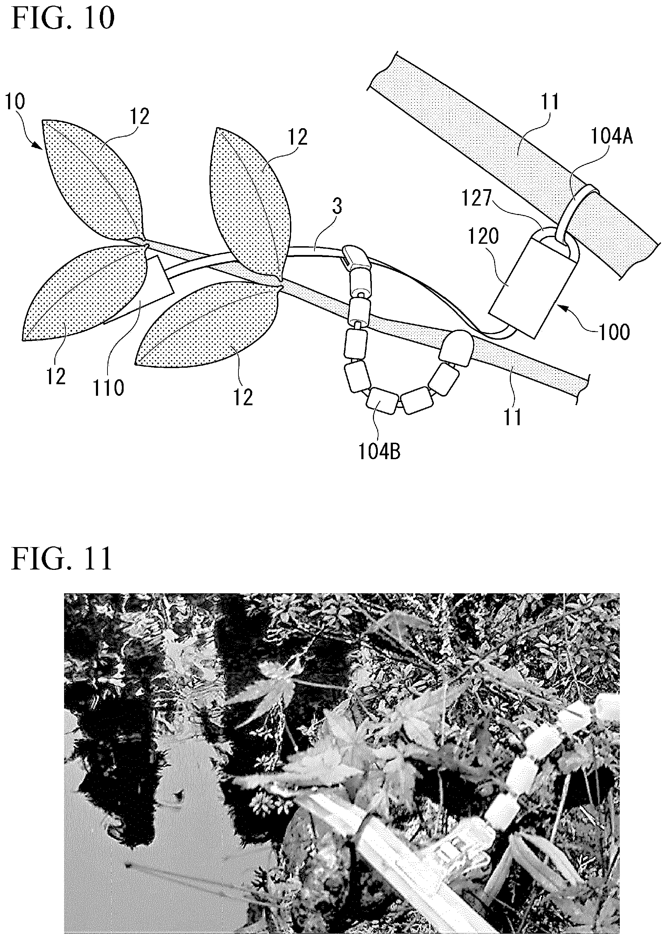

FIG. 10 is a perspective view showing an example in which the system 100 of the embodiment is attached to the plant 10.

Referring to FIG. 10 together, a branch-and-stem side housing 120 constituting the system 100 is fixed to the branch 11 or the stem. In the example shown in the figure, the branch-and-stem side housing 120 is fixed to the branch 11 by a binding band 104A (an example of the branch side fixing portion) being engaged with the U-shaped first engagement portion 127. The fixation aspect of the branch-and-stem side housing 120 is not limited to the above and can be changed according to the design specifications.

For example, the leaf side housing 110 constituting the system 100 is fixed to the back surface 14 of the leaf 12. In the example shown in the figure, the leaf side housing 110 is fixed by being adhered to the back surface 14 of the leaf 12 using double-sided tape (not shown) (an example of a leaf side fixing portion) that is attached to the periphery of the slit 113. The fixation aspect of the leaf side housing 110 is not limited to the above and can be changed according to the design specifications.

For example, a part of the support 3 constituting the system 100 is fixed to the branch 11 or the stem. In the example shown in the figure, a portion of the support 3 between the leaf side housing 110 and the branch-and-stem side housing 120 is fixed to the branch 11 by an articulated clip 104B (an example of the branch side fixing portion). The fixation aspect of the support 3 is not limited to the above and can be changed according to the design specifications.

### <Operations and effects>

As described above, the system 100 according to the present embodiment includes a part of the support structure 1, a light source 107 that is supported on a portion of the support 3 which is disposed on a side of the back surface 14 of the leaf 12, a leaf side housing 110 that accommodates a portion of the support 3 which is disposed on a side of the back surface 14 of the leaf 12 together with an acquisition unit 102 and the light source 107, a control device 130 that controls the acquisition unit 102 and the light source 107, a power source 131 that supplies power to the acquisition unit 102, the light source 107, and the control device 130, and a branch-and-stem side housing 120 that accommodates a portion of the support 3 which is disposed on a side of the branch 11 or the stem together with the control device 130 and the power source 131 and is fixed to the branch 11 or the stem.

According to this configuration, since the acquisition unit 102 that constitutes the support structure 1 is disposed on a side of the back surface 14 of the leaf 12 rather than on a side of the front surface 13 of the leaf 12, there is no need to provide a structure on a side of the front surface 13 of the leaf 12. For this reason, damage to the leaf 12 of the plant 10 or inhibition of photosynthesis during the long-term observation of the leaf 12 can be suppressed. In addition, since the support 3 that supports the acquisition unit 102 is fixed by the fixing portion in a state in which the support 3 extends from a side of the back surface 14 of the leaf 12 to the branch 11 of the plant 10, robustness of attaching capability can be maintained. Therefore, it is possible to provide the system 100 by which the damage to the leaf 12 of the plant 10 or the inhibition of the photosynthesis during the long-term observation of the leaf 12 can be suppressed and the robustness of the attaching capability can be maintained.

Furthermore, by providing the power source 131 that supplies power to the acquisition unit 102, the light source 107, and the control device 130, it is possible to perform the long-term observation of the leaf 12 of the plant 10 without the need for an external power source. In addition, by providing the leaf side housing 110 that accommodates a portion of the support 3 which is disposed on a side of the back surface 14 of the leaf 12 together with the acquisition unit 102 and the light source 107, it is possible to protect the acquisition unit 102 and the light source 107 from external factors. In addition, by providing the branch-and-stem side housing 120 that accommodates a portion of the support 3 which is disposed on a side of the branch 11 or the stem together with the control device 130 and the power source 131, it is possible to protect the control device 130 and the power source 131 from external factors. In addition, by fixing the branch-and-stem side housing 120 to the branch 11 or the stem, the robustness of the attaching capability can be maintained during the long-term observation of the leaf 12 of the plant 10.

In the system 100 according to the present embodiment, the branch-and-stem side housing 120 is fixed to the branch 11 with the binding band 104A, the leaf side housing 110 is fixed to the back surface 14 of the leaf 12 with the double-sided tape, and a part of the support 3 is fixed to the branch 11 with the articulated clip 104B.

According to this configuration, the weight of the support 3 is dispersed to a side of the leaf 12 and a side of the branch 11, and thus it is possible to prevent the plant 10 from being damaged. In addition, in the system 100, the branch-and-stem side housing 120 (a structure including the control device 130, the power source 131, and the like), which is a heavy object, is fixed to the branch 11 with the binding band, and thus the robustness of the attaching capability can be sufficiently maintained.

The leaf side housing 110 according to the present embodiment includes the upper cover 111 and the lower cover 112. The joint portion between the upper cover 111 and the lower cover 112 is sealed with a sealing material.

According to this configuration, it is possible to prevent rainwater and the like from entering the internal space of the leaf side housing 110 through the joint portion between the upper cover 111 and the lower cover 112. Therefore, it is possible to maintain a waterproofing property sufficient to withstand the long-term outdoor measurements.

The branch-and-stem side housing 120 according to the present embodiment includes the upper case 121 and the lower case 122. The joint portion between the upper case 121 and the lower case 122 is sealed with a sealing material.

According to this configuration, it is possible to prevent rainwater and the like from entering the internal space of the branch-and-stem side housing 120 through the joint portion between the upper case 121 and the lower case 122. Therefore, it is possible to maintain a waterproofing property sufficient to withstand the long-term outdoor measurements.

In the above embodiment, an example in which the system is applied to the measurement system for outdoor use which is powered by the battery that is the power source has been described, but the present invention is not limited to this. For example, the system may be applied to a measurement system for indoor use (for example, for use in a greenhouse, a plant factory, or the like) which is powered by an external power source. For example, the application aspect of the system can be changed according to the design specifications.

For example, in the above embodiment, an example in which the system includes the power source that is accommodated in the branch-and-stem side housing and supplies power to the acquisition unit, the light source, and the control device has been described, but the present invention is not limited to this. For example, the power source does not need to be accommodated in the branch-and-stem side housing. For example, the system may be formed such that it is possible to supply power to the acquisition unit, the light source, and the control device from an external power source installed in a greenhouse, a plant factory, or the like. For example, power may be supplied to the control device or the like within the housing via an electric wire connected to the external power source. For example, the installation aspect of the power source can be changed according to the design specifications.

**In** addition, it is possible to replace the constituent elements in the above-described embodiments with well-known constituent elements without departing from the spirit of the present invention. **In** addition, the above-mentioned modification examples may be combined with each other.

### [Example]

The system 100 according to the above embodiment of the present invention will be specifically described below by showing an example. The following example is a specific example to which the present invention is applied and is not intended to limit the present invention.

### <Example>

FIG. 11 is a perspective view showing an example in which a system of the example is attached to a plant.

Referring to FIG. 11, as the system of the example, a system which includes a support that is a part of the support structure, a light source that is supported on a portion of the support which is disposed on a side of the back surface of the leaf, a leaf side housing that accommodates a portion of the support which is disposed on a side of the back surface of the leaf together with an acquisition unit and the light source, a control device that controls the acquisition unit and the light source, a power source that supplies power to the acquisition unit, the light source, and the control device, and a branch-and-stem side housing that accommodates a portion of the support which is disposed on a side of the branch together with the control device and the power source and is fixed to the branch and in which the branch-and-stem side housing is fixed to the branch with the binding band, the leaf side housing is fixed to the back surface of the leaf with the double-sided tape, and a part of the support is fixed to the branch with articulated clip (corresponding to the configuration of the system 100 shown in FIGS. 4 to 6 and 10) was prepared. The plant to which the system of the example is to be attached is a maple tree.

### <Observation results>

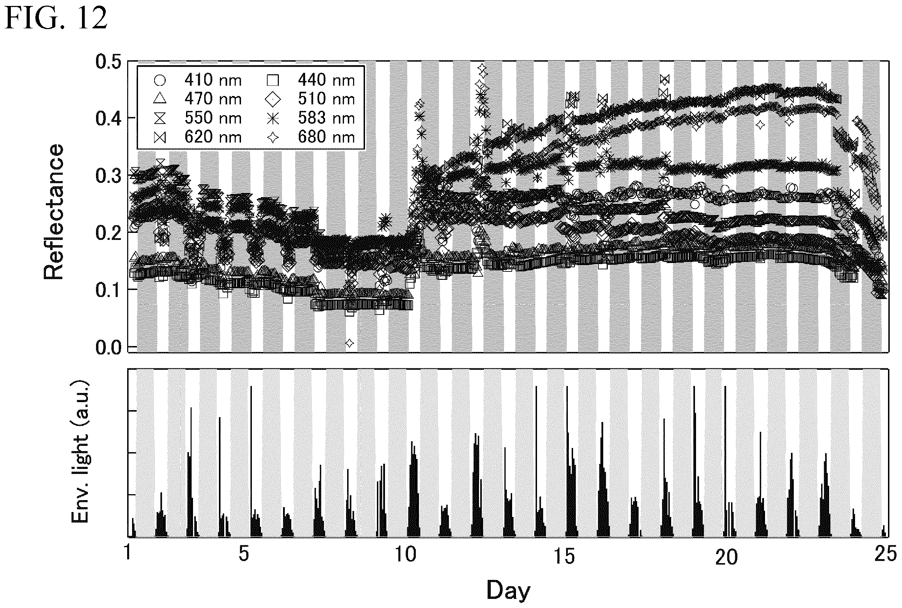

Using the system of the example, the leaf of the maple tree was observed outdoors for a long period of time. FIG. 12 is a graph showing measurement data obtained by the system of the example, in which the upper part of FIG. 12 shows reflection spectrum data, and the lower part of FIG. 12 shows the ambient light. The upper part of FIG. 12 shows the transition of reflectance at each wavelength as the reflection spectrum data, and the lower part of FIG. 12 shows the sunlight shining that passes through the leaf as the ambient light (corresponding to the amount of sunlight).

As shown in FIG. 12, the system of the example was able to acquire data on a change in light reflectance according to a change in color of the leaf of the maple tree. In addition, it was also confirmed that the reflectance of light changes according to each wavelength.

For example, by confirming a change in reflectance according to the color of the leaf of the plant or a change in reflectance according to each wavelength, it is possible to understand how the physiological response of the plant changes over time. In addition, it is also known that by measuring light reflection, it is possible to monitor (observe and survey) disease, stress, a growth state, and the like, and it is suggested that this technology can be used to remotely monitor a health state of agricultural crops.

### BRIEF DESCRIPTION OF THE REFERENCE SYMBOLS

1 Support structure
2A, 2B Leaf side acquisition unit (acquisition unit)
2C Outer side acquisition unit (acquisition unit)
3 Support
4A, 4B Leaf side fixing portion (wall side fixing portion, fixing portion)
4C Branch side fixing portion (branch-and-stem side fixing portion, fixing portion)
5 Barrier
6 Interposition portion
10 Plant
11 Branch
12 Leaf
13 Front surface of leaf
14 Back surface of leaf
15 Main vein
100 System
102 Acquisition unit
107 Light source
110 Leaf side housing
120 Branch-and-stem side housing
130 Control device
131 Power source

## Claims

1. A support structure for arranging an acquisition unit, which acquires information on a state of a leaf of a plant or information on a surrounding environment, in the vicinity of the leaf, comprising:
a support that supports the acquisition unit; and
a fixing portion that fixes the support to the plant,
wherein the support is fixed by the fixing portion in a state in which the support extends from a side of a back surface of the leaf to a branch or a stem of the plant such that the acquisition unit is disposed on a side of the back surface of the leaf rather than on a side of a front surface of the leaf.

2. The support structure according to claim 1, wherein the support has elasticity.

3. The support structure according to claim 1 or 2,
wherein a plurality of acquisition units are provided, and
wherein the plurality of acquisition units are fixed to the support at different positions in a location at which the support overlaps the leaf.

4. The support structure according to claim 1 or 2,
wherein the support extends from a location at which the support overlaps the leaf to a location at which the support does not overlap the leaf,
wherein a plurality of acquisition units are provided, and
wherein the plurality of acquisition units include
a leaf side acquisition unit that is fixed to the support in a location at which the support overlaps the leaf, and
an outer side acquisition unit that is fixed to the support in a location at which the support does not overlap the leaf.

5. The support structure according to claim 1 or 2,
wherein a plurality of fixing portions are provided, and
wherein the plurality of fixing portions include
a leaf side fixing portion that fixes a portion of the support which is disposed on a side of the back surface of the leaf to the back surface of the leaf, and
a branch-and-stem side fixing portion that fixes a portion of the support which is disposed on a side of the branch or the stem to the branch or the stem.

6. The support structure according to claim 1 or 2, further comprising a barrier that is fixed to the support and surrounds the acquisition unit to provide a space between the support and the back surface of the leaf.

7. The support structure according to claim 6, further comprising a wall side fixing portion that fixes at least a part of a portion of the barrier on a side opposite to the support to the back surface of the leaf.

8. The support structure according to claim 7, wherein an area of the wall side fixing portion is smaller than an area of the entire portion of the barrier on a side opposite to the support.

9. The support structure according to claim 7, further comprising an interposition portion that is interposed to fill a gap between a portion of the barrier on a side opposite to the support and the back surface of the leaf.

10. The support structure according to claim 9, wherein the interposition portion has a light blocking property.

11. The support structure according to claim 1 or 2, wherein the support extends along a main vein on a side of the back surface of the leaf and then extends toward the branch or the stem of the plant.

12. The support structure according to claim 1 or 2, wherein an area of a portion of the support which is disposed on a side of the back surface of the leaf and is fixed to the back surface of the leaf is smaller than an area of the entire back surface of the leaf.

13. A system comprising:
at least a part of the support structure according to claim 1 or 2;
a light source that is supported on a portion of the support which is disposed on a side of the back surface of the leaf;
a leaf side housing that accommodates a portion of the support which is disposed on a side of the back surface of the leaf together with the acquisition unit and the light source;
a control device that controls the acquisition unit and the light source; and
a branch-and-stem side housing that accommodates a portion of the support which is disposed on a side of the branch or the stem together with the control device and is fixed to the branch or the stem.
